# EUROPÄISCHE PATENTSCHRIFT

(11) **EP 1 565 234 B1**
(45) Veröffentlichungstag und Bekanntmachung des Hinweises auf die Patenterteilung: **27.09.2006**
(21) Anmeldenummer: 03767564.2
(22) Anmeldetag: 17.11.2003
(51) Int. Cl.: A61N 1/39

(54) **DEFIBRILLATOR MIT EINER RELAIS-TESTEINRICHTUNG**
DEFIBRILLATOR COMPRISING A RELAY TESTING DEVICE
DEFIBRILLATEUR MUNI D UN DISPOSITIF D'ESSAI DE RELAIS

(30) Priorität: 19.11.2002 DE 10254482
(43) Veröffentlichungstag der Anmeldung: 24.08.2005
(73) Patentinhaber: Metrax GmbH, D-78628 Rottweil (DE)
(72) Erfinder: BUCHER, Heinz, 78615 Rottweil (DE)
(74) Vertreter: Fleck, Hermann-Joseph
(86) Internationale Anmeldenummer: PCT/EP2003/012855
(87) Internationale Veröffentlichungsnummer: WO 2004/045712

(56) Entgegenhaltungen:
- EP-A- 0 946 956
- US-A- 5 097 830
- US-A- 5 275 158
- US-A- 5 431 684
- US-A- 5 879 374

## Beschreibung

Die Erfindung bezieht sich auf einen Defibrillator mit einer Endstufe, die einen Hochspannungsteil und mit diesem mittels einer Ankoppelschaltung automatisch über ein Relais verbindbare Patientenelektroden-Anschlüsse sowie eine Relais-Testeinrichtung aufweist, und zwar insbesondere einen tragbaren externen Defibrillator.

Ein derartiger Defibrillator ist in der EP 0 946 956 B1 angegeben. Bei diesem bekannten Defibrillator ist ein Verfahren vorgesehen, mit dem ein Relais getestet wird, das in einer Ankoppelschaltung zwischen einem Hochspannungsteil und Patientenelektroden angeordnet ist und einerseits in eine offene Stellung und andererseits in eine geschlossene Stellung, in der die Patientenelektroden mit dem Hochspannungsteil verbunden sind, bringbar ist. Zum Testen des Relais wird ein Testablauf durchgeführt, bei dem eine Entladung eines Energiespeichers in Form eines die Energie für den Defibrillationsimpuls speichernden Kondensators über das Relais und die Patientenelektroden vorgenommen und die Spannung an dem Kondensator gemessen wird. Die Spannung des Kondensators während der Entladung wird mit einer Schwellenspannung verglichen, um auf den Relaiszustand zurückzuschließen. Es ist schwierig, auf diese Weise in unterschiedlichen Situationen eine zuverlässige Aussage über den Relaiszustand herzuleiten.

Ein insbesondere tragbarer automatischer externer Defibrillator (AED) mit einem besonders ausgebildeten Hochspannungsteil mit H-Brücke und in deren Querzweig in Reihe zu einer Induktivität (Spule oder äquivalentes Bauteil) angeschlossenen Patientenelektroden ist in der DE 100 65 104 A1 angegeben. Bei diesem bekannten Defibrillator können vorteilhaft biphasische Defibrillationsimpulse durch entsprechende Steuerung von Schaltgliedern der H-Brücke erzeugt werden. Ein Relaistest ist dabei nicht angegeben.

Die US 5,879,374 offenbart einen Defibrillator gemäß dem Oberbegriff des Anspruchs 1. Bei diesem Defibrillator ist eine Relais-Testeinrichtung vorhanden, die jedoch nicht den Zustand der Entladewiderstandsanordnung einbezieht.

Der Erfindung liegt die Aufgabe zugrunde, einen insbesondere tragbaren externen Defibrillator der eingangs genannten Art bereit zu stellen, mit dem die Prüfung des Ankoppelrelais möglichst zuverlässig durchführbar ist.

Diese Aufgabe wird mit den Merkmalen des Anspruches 1 gelöst. Hiernach ist vorgesehen, dass eine Entladewiderstandsanordnung vorhanden ist, auf die mittels des Relais anstelle der Patientenelektroden-Anschlüsse automatisch umschaltbar ist und dass die Relais-Testeinrichtung zum Testen des Relais unter Einbeziehung des Zustandes der anliegenden Entladewiderstandsanordnung ausgebildet ist.

Durch die angeschlossene Entladewiderstandsanordnung werden zuverlässige Bedingungen zum Testen des Relais geschaffen, so dass eine zuverlässige Auswertung des Relaiszustandes erreicht wird.

Die zuverlässige Prüfung des Ankoppel-Relais wird dadurch begünstigt, dass die Relais-Testeinrichtung eine eigene Spannungsversorgung für eine Test-Versorgungsspannung aufweist, mit der zum Testen des Relais bei angeschlossener Entladewiderstandsanordnung ein Strom durch das Relais bewirkbar ist, wobei ein Strom von dem Hochspannungsteil unterbunden ist. Diese Maßnahmen tragen zu eindeutigen Testbedingungen bei, wobei ein einfacher Aufbau der Messeinrichtung realisierbar ist.

Eine weitere vorteilhafte Ausgestaltung der Relais-Testeinrichtung besteht darin, dass sie einen Messzweig aufweist, in dem bei anliegender Entladewiderstandsanordnung einerseits und bei nicht anliegender Entladewiderstandsanordnung andererseits unterschiedliche Spannungen oder Messströme anliegen, die beim Testen des Relais einbeziehbar sind.

Hierbei wird eine zuverlässige Messung dadurch unterstützt, dass der Messzweig eine Messverstärkerschaltung zum Bilden eines Messwerts für einen Relaiszustand aufweist.

Eindeutige Aussagen werden ferner dadurch begünstigt, dass die Messverstärkerschaltung einen Komparator zum Vergleichen mit einem Sollwert aufweist.

Ein vorteilhafter Aufbau des Defibrillators mit der Relais-Testeinrichtung besteht ferner darin, dass der Hochspannungsteil eine von einem Energiespeicher mit Hochspannung für einen Defibrillationsimpuls beaufschlagbare H-Brücke mit steuerbaren Schaltgliedern in den H-Schenkeln aufweist und dass das Relais in Reihe mit einer Induktivität und einerseits dem Entladewiderstand oder andererseits den an die Patientenelektroden-Anschlüsse angeschlossenen Patientenelektroden in dem Querzweig der H-Brücke angeordnet ist.

Eine weitere vorteilhafte Ausgestaltung des Defibrillators mit der Relais-Testeinrichtung besteht darin, dass zwischen dem Relais und den Patientenelektroden-Anschlüssen ein weiteres Relais eingebunden ist, mit dem die Patientenelektroden wahlweise mit dem Hochspannungsteil oder einer EKG-Messeinrichtung verbindbar sind.

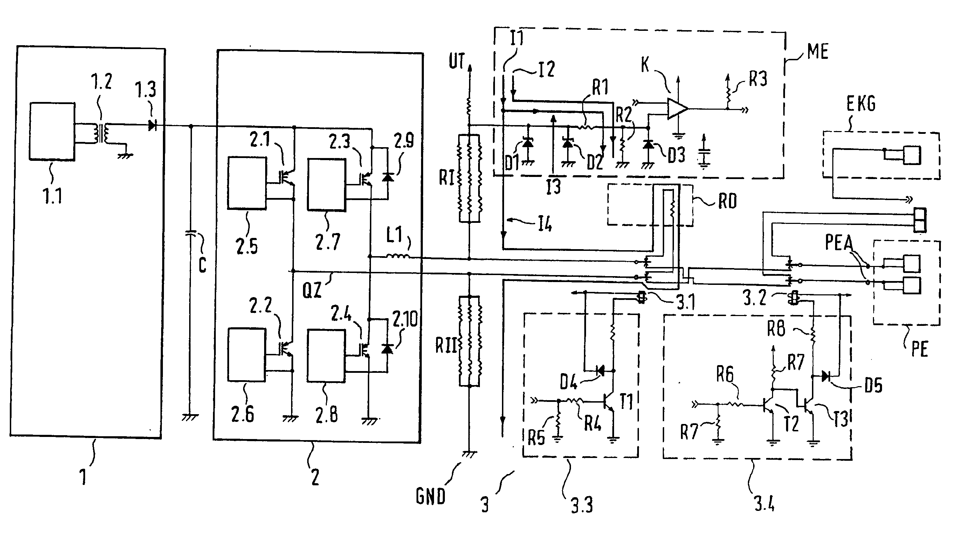

Die Erfindung wird nachfolgend anhand eines Ausführungsbeispiels unter Bezugnahme auf die Zeichnung näher erläutert.

Die Fig. zeigt eine Endstufe für einen Defibrillator, insbesondere einen tragbaren externen automatischen Defibrillator (AED), mit einem eine Ladeeinrichtung 1 und eine H-Brücke 2 aufweisenden Hochspannungsteil sowie einer daran angeschlossenen Ankoppelschaltung 3. Die Ladeeinrichtung weist einen Ladeteil 1.1, beispielsweise eine Wechselspannungsquelle, mit daran angeschlossenem Transformator 1.2 zum Erzeugen einer Hochspannung auf, mit der über eine Ladediode 1.3 in an sich bekannter Weise ein Energiespeicher in Form eines Speicherkondensators C oder einer Speicherkondensatoranordnung mit elektrischer Energie für die Defibrillation geladen wird. Die H-Brücke 2 weist in ihren zu dem Pluspol einerseits und dem Minuspol andererseits gerichteten H-Schenkeln jeweilige Schaltglieder 2.1, 2.2, 2.3, 2.4 auf, die mittels zugeordneter Ansteuerschaltungen 2.5, 2.6, 2.7, 2.8 angesteuert werden, wie beispielsweise in der eingangs genannten DE 100 65 104 A1 näher beschrieben. Als Besonderheit ist bei vorliegender H-Brücke 2 antiparallel zu zwei Schaltgliedern 2.3, 2.4, von denen eines in einem zu dem Pluspol führenden H-Schenkel und eines in dem gegenüberliegenden, zum Minuspol führenden H-Schenkel angeordnet ist, jeweils eine Diodenanordnung 2.9, 2.10 angeordnet, um eine zuverlässige Funktion der Schaltglieder 2.1, 2.2, 2.3, 2.4 bei biphasischem Betrieb durch Freilauf zu gewährleisten, insbesondere wenn Schaltvorgänge höherer Frequenz von z.B. 10 kHz oder mehr zur Regelung der Pulsenergie beispielsweise mittels Stromregelung durchgeführt werden. In dem Querzweig QZ der H-Brücke 2 liegt eine Induktivität L1 in Form einer Spule oder eines äquivalenten Schaltungsteils in Reihe zu einem Ankoppel-Relais 3.1 der Ankoppelschaltung 3 sowie in Reihe zu einem Entladewiderstand RD, oder wahlweise zu diesem mittels des Relais automatisch anschließbaren Patientenelektroden-Anschlüssen PEA, an die im Betrieb Patientenelektroden PE angeschlossen sind. Bei dem Entladewiderstand RD handelt es sich beispielsweise um einen der Patientenimpedanz vergleich-baren Widerstand mit einem definierten Wert z.B. im Bereich zwischen 10 und 100 Ohm, beispielsweise 25 oder 50 Ohm. Über den Entladewiderstand kann der Energiespeicher C nach einer festlegbaren Zeit des Nichtgebrauchs oder nach einer Defibrillation zum Gewährleisten eines definierten Zustandes entladen werden.

Die Ankoppelschaltung 3 weist außer dem Relais 3.1 ein zwischen diesem und den Patientenelektroden-Anschlüssen PEA liegendes weiteres Relais 3.2 auf, mit dem die Patientenelektroden PE wahlweise mit einer EKG-Messeinrichtung EKG oder der H-Brücke 2 des Hochspannungsteils verbindbar sind. Beide Relais 3.1, 3.2 sind über jeweils zugeordnete Relaisansteuerungen 3.3 bzw. 3.4 ansteuerbar, wobei die Relaisansteuerung 3.3 bei dem gezeigten Ausführungsbeispiel Steuerkreis-Widerstände R4, R5, einen Steuerkreis-Transistor T1 sowie eine Steuerkreis-Diode D4 in gezeigter Anordnung aufweist. Die weitere Relaisansteuerung 3.4 weist Steuerkreis-Widerstände R6, R7, R8, zwei Steuerkreis-Transistoren T2, T3 sowie eine Steuerkreis-Diode in gezeigter Anordnung auf.

Mit den Relaisansteuerungen 3.3, 3.4, die auch in anderer Weise aufgebaut sein können, können die beiden Relais 3.1, 3.2 zwischen den gezeigten Schaltstellungen umgesteuert werden.

Weiterhin ist die Ankoppelschaltung 3 mit einer Relais-Testeinrichtung für das Ankoppel-Relais 3.1 ausgestattet. Die Relais-Testeinrichtung dient zum Prüfen des Relais 3.1, wobei der Hochspannungsteil, und zwar vorliegend die H-Brücke 2 durch entsprechende Ansteuerung der Schaltglieder 2.1, 2.2, 2.3, 2.4 stromlos geschaltet ist. Stattdessen wird eine für den Relais-Test gesondert vorgesehene Test-Versorgungsspannung UT von einer Test-Versorgungsspannungseinrichtung in der Größenordnung einiger oder einiger 10 Volt, z.B. zwischen 6 und 30 Volt bereitgestellt. Die Relais-Testeinrichtung ist so aufgebaut, dass bei an das Relais 3.1 angeschlossenem Entladewiderstand RD ein geschlossener Stromkreis von dem Pluspol der Testversorgungsspannung UT über eine erste Widerstandsanordnung RI mit mehreren Widerständen über den Entladewiderstand RD, die entsprechenden Kontakte des Relais 3.1 und eine weitere Widerstandsanordnung RII mit mehreren Widerständen nach Masse GND gebildet ist. Parallel zu diesem Strompfad ist zwischen dem Pluspol der Test-Versorgungsspannung UT und der ersten Widerstandsanordnung RI ein Messzweig ME mit Messkreis-Widerständen R1, R2, R3 und Messkreis-Dioden D1, D2, D3 und einer Komparatorschaltung K gebildet, wobei der Messkreis-Widerstand R1 in einem zu einem Eingangsanschluss der Komparatorschaltung K führenden Strompfad liegt, der Messkreis-Widerstand R2 zwischen dem ersten Messkreis-Widerstand R1 und dem Eingangsanschluss der Komparatorschaltung K gegen Masse geführt ist und der dritte Messkreis-Widerstand R3 am Ausgang der Komparatorschaltung K zu einem vorgegebenen Potential geführt ist. Die Messkreis-Dioden D1, D2 sind als stabilisierende Zener-Dioden ausgeführt und vor dem ersten Messkreis-Widerstand R1 gegen Masse geführt, während die dritte Messkreis-Diode D3 parallel zu dem zweiten Messkreis-Widerstand R2 nach Masse geführt ist. An dem zweiten Eingangsanschluss der Komparatorschaltung K liegt eine vorgegebene oder vorgebbare Vergleichsspannung.

Ist der Entladewiderstand RD mit dem Relais 3.1 in dem betreffenden Strompfad angeschlossen, fließt von einem von dem Pluspol der Testversorgungsspannung UT bis zu dem Abzweigpunkt des Messzweiges ME vorliegenden Gesamtstrom I1 ein Teil-Strom I4 durch das Relais 3.1, während ein weiterer Teilstrom als Messstrom I3 durch den Messzweig ME über den zweiten Messkreis-Widerstand R2 nach Masse fließt und an diesem einen entsprechenden Spannungsabfall erzeugt, der im angeschlossenen Zustand des Relais 3.1 eine diesem Zustand entsprechende Vergleichsspannung ergibt.

Ist der Entladewiderstand RD durch entsprechende Ansteuerung des Relais 3.1 nicht angeschlossen, fließt der Gesamtstrom I1 als Messstrom I2 durch den Messzweig ME und erzeugt an dem zweiten Messkreis-Widerstand R2 einen entsprechend höheren Spannungsabfall, der mittels der Komparatorschaltung K feststellbar ist. Mit dem Messzweig ME kann also zwischen einem angeschlossenen und nicht angeschlossenen Zustand des Entladewiderstandes RD unterschieden und somit auf die ordnungsgemäße Funktion des Relais 3.1 rückgeschlossen werden, wozu das Ausgangssignal der Komparatorschaltung K in einer Auswerteschaltung geeignet ausgewertet wird.

Auch andere Verstärkerschaltungen in dem Messzweig ME sind denkbar, wobei beispielsweise auch nicht vollständig geschlossene und vollständig offene Zustände des Relais 3.1 unterschieden werden können, soweit eine derartige Auswertung erwünscht ist. Wegen der gesonderten Test-Versorgungsspannung UT ist die Relais-Testeinrichtung unabhängig von dem Hochspannungsteil, so dass eine zuverlässige Aussage über die Relais-Funktionsfähigkeit ermöglicht wird. Die Steuerschaltung des Defibrillators ist so ausgebildet, dass sie nur bei ordnungsgemäßer Funktion des Relais 3.1 den Hochspannungsteil mit den Patientenelektroden PE verbindet. Diese Funktion kann dann beispielsweise auch dadurch realisiert werden, dass das weitere Relais 3.2 so angesteuert wird, dass dieses die Verbindung zwischen den Patientenelektroden PE bzw. den Patientenelektroden-Anschlüssen PEA mit dem Hochspannungsteil unterbricht, wenn ein Fehlerzustand des Koppel-Relais 3.1 festgestellt wird. Die Auswertung kann in einer geeigneten Logikschaltung, z.B. einem programmierten Mikrocontroller oder einer anderen programmierbaren Logikeinheit (CPLD) erfolgen und für weitere Steueraufgaben genützt werden.

## Patentansprüche

1. Defibrillator mit einer Endstufe, die einen Hochspannungsteil (1, 2) und mit diesem mittels einer Ankoppelschaltung (3) automatisch über ein Relais (3.1) verbindbare Patientenelektroden-Anschlüsse (PEA) sowie eine Relais-Testeinrichtung aufweist,
wobei eine Entladewiderstandsanordnung (RD) vorhanden ist, auf die mittels des Relais (3.1) anstelle der Patientenelektroden-Anschlüsse (PEA) automatisch umschaltbar ist, **dadurch gekennzeichnet, dass** die Relais-Testeinrichtung zum Testen des Relais (3.1) unter Einbeziehung des Zustandes der anliegenden Entladewiderstandsanordnung (RD) ausgebildet ist.

2. Defibrillator nach Anspruch 1,
**dadurch gekennzeichnet,**
**dass** die Relais-Testeinrichtung eine eigene Spannungsversorgung für eine Test-Versorgungsspannung (UT) aufweist, mit der zum Testen des Relais (3.1) bei angeschlossener Entladewiderstandsanordnung (RD) ein Strom (I4) durch das Relais (3.1) bewirkbar ist, wobei ein Strom von dem Hochspannungsteil (1, 2) her unterbunden ist.

3. Defibrillator nach Anspruch 1 oder 2,
**dadurch gekennzeichnet, dass** die Relais-Testeinrichtung einen Messzweig (ME) aufweist, in dem bei anliegender Entladewiderstandsanordnung (RD) einerseits und bei nicht anliegender Entladewiderstandsanordnung (RD) andererseits unterschiedliche Spannungen oder Messströme (I₃, I₂) anliegen, die beim Testen des Relais (3.1) einbeziehbar sind.

4. Defibrillator nach Anspruch 3,
**dadurch gekennzeichnet, dass** der Messzweig (ME) eine Messverstärkerschaltung zum Bilden eines Messwerts für einen Relaiszustand aufweist.

5. Defibrillator nach Anspruch 4,
**dadurch gekennzeichnet, dass** die Messverstärkerschaltung einen Komparator zum Vergleichen mit einem Sollwert aufweist.

6. Defibrillator nach einem der vorhergehenden Ansprüche,
**dadurch gekennzeichnet,**
**dass** der Hochspannungsteil eine von einem Energiespeicher (C) mit Hochspannung für einen Defibrillationsimpuls beaufschlagbare H-Brücke (2) mit steuerbaren Schaltgliedern (2.1, 2.2, 2.3, 2.4) in den H-Schenkeln aufweist und
**dass** das Relais (3.1) in Reihe mit einer Induktivität (L1) und einerseits dem Entladewiderstand (RD) oder andererseits den an die Patientenelektroden-Anschlüsse (PEA) angeschlossenen Patientenelektroden (PE) in dem Querzweig (QZ) der H-Brücke (2) angeordnet ist.

7. Defibrillator nach einem der vorhergehenden Ansprüche,
**dadurch gekennzeichnet,**
**dass** zwischen dem Relais (3.1) und den Patientenelektroden-Anschlüssen (PEA) ein weiteres Relais (3.2) eingebunden ist, mit dem die Patientenelektroden (PE) wahlweise mit dem Hochspannungsteil (1, 2) oder einer EKG-Messeinrichtung (EKG) verbindbar sind.

## Claims

1. Defibrillator having an output stage which has a high-voltage element (1, 2) and patient electrode connectors (PEA), which can be automatically connected to said element by means of a coupling circuit (3) via a relay (3.1), as well as a relay testing device,
a discharge resistor arrangement (RD) being present to which it is possible to switch automatically by means of the relay (3.1) instead of the patient electrode connectors (PEA), **characterised in that** the relay testing device is designed for testing the relay (3.1) while incorporating the status of the connected discharge resistor arrangement (RD).

2. Defibrillator according to claim 1, **characterised in that** the relay testing device has its own voltage supply for a test supply voltage (UT) with which a current (14) can be run through the relay (3.1) for testing the relay with the discharge resistor arrangement (RD) connected, a current from the high-voltage element (1, 2) being blocked.

3. Defibrillator according to claim 1 or 2, **characterised in that** the relay testing device has a measuring branch (ME) in which, when the discharge resistor (RD) is connected on the one hand and disconnected on the other hand, different voltages or measuring currents (I₃, I₂) are present which can be incorporated in the testing of the relay (3.1).

4. Defibrillator according to claim 3, **characterised in that** the measuring branch (ME) has a measurement amplification circuit for forming a measured value for a relay status.

5. Defibrillator according to claim 4, **characterised in that** the measurement amplification circuit has a comparator for comparing with a desired value.

6. Defibrillator according to one of the preceding claims, **characterised in that** the high-voltage element has an H-bridge (2) which is chargeable by an energy storage device (C) with high voltage for a defibrillation pulse and has controllable switching members (2.1, 2.2, 2.3, 2.4) in the H-legs, and **in that** the relay (3.1) is arranged in series with an inductive resistor (L1) and on the one hand with the discharge resistor (RD) or on the other hand with the patient electrodes (PE) connected to the patient electrode connectors (PEA) in the transverse branch (QZ) of the H-bridge (2).

7. Defibrillator according to one of the preceding claims, **characterised in that** there is integrated between the relay (3.1) and the patient electrode connectors (PEA) a further relay (3.2) with which the patient electrodes (PE) can be optionally connected to the high-voltage element (1, 2) or an ECG measuring device (EKG).

## Revendications

1. Défibrillateur avec un étage terminal qui comprend une partie à haute tension (1, 2) et des raccords d'électrodes à appliquer au patient (PEA), lesquels peuvent être reliés automatiquement avec celle-ci au moyen d'un circuit de couplage (3) et par l'intermédiaire d'un relais (3.1), ainsi qu'un dispositif de test de relais,
un ensemble de résistances de décharge (RD), sur lequel peut avoir lieu une commutation automatique au moyen du relais (3.1) au lieu de la commutation sur les raccords d'électrodes à appliquer au patient (PEA), étant disponible,
**caractérisé**
**en ce que** le dispositif de test de relais servant à tester le relais (3.1) est configuré en incluant l'état de l'ensemble de résistances de décharge (RD) qui est disponible.

2. Défibrillateur selon la revendication 1,
**caractérisé**
**en ce que** le dispositif de test de relais comprend une alimentation en tension propre fournissant une tension d'alimentation de test (UT) avec laquelle peut être obtenu un courant (14) traversant le relais (3.1) pour tester ce relais (3.1) lorsque l'ensemble de résistances de décharge (RD) est raccordé, un courant provenant de la partie à haute tension (1, 2) étant bloqué.

3. Défibrillateur selon la revendication 1 ou 2,
**caractérisé**
**en ce que** le dispositif de test de relais comprend une branche de mesure (ME) sur laquelle, d'une part lorsque l'ensemble de résistances de décharge (RD) est raccordé et d'autre part lorsque l'ensemble de résistances de décharge (RD) n'est pas raccordé, sont présents des tensions ou des courants de mesure différents (I₃, I₂) qui peuvent être pris en considération lors du test du relais (3.1).

4. Défibrillateur selon la revendication 3,
**caractérisé**
**en ce que** la branche de mesure (ME) comprend un circuit d'amplification de mesure pour former une valeur de mesure pour un état du relais.

5. Défibrillateur selon la revendication 4,
**caractérisé**
**en ce que** le circuit d'amplification de mesure comprend un comparateur pour réaliser une comparaison avec une valeur de consigne.

6. Défibrillateur selon l'une quelconque des revendications précédentes,
**caractérisé**
**en ce que** la partie à haute tension comprend un pont en H (2) auquel une haute tension peut être appliquée par un accumulateur d'énergie (C) pour générer une impulsion de défibrillation et qui comprend dans les branches en H des éléments de commutation pouvant être commandés (2.1, 2.2, 2.3, 2.4)
et
**en ce que** le relais (3.1) est disposé dans la branche transversale (QZ) du pont en H (2) en étant commuté en série avec une inductance (L1) et d'une part avec la résistance de décharge (RD) ou d'autre part avec les électrodes à appliquer au patient (PE) qui sont branchées sur les raccords d'électrodes à appliquer au patient (PEA).

7. Défibrillateur selon l'une quelconque des revendications précédentes,
**caractérisé**
**en ce qu'**entre le relais (3.1) et les raccords d'électrodes à appliquer au patient (PEA) est placé un autre relais (3.2) avec lequel les électrodes à appliquer au patient (PE) peuvent au choix être reliées avec la partie à haute tension (1, 2) ou avec un électrocardiographe (ECG).
